# EUROPEAN PATENT APPLICATION

(11) **EP 4 553 061 A1**
(43) Date of publication of application: **14.05.2025**
(21) Application number: 23835530.9
(22) Date of filing: 04.07.2023
(51) Int. Cl.: C07C 41/22, C07C 43/12, C07C 43/225, C07C 319/20, C07C 323/12, C07C 67/307, C07C 69/716

(54) **METHOD FOR PRODUCING FLUORINATED ORGANIC COMPOUND**

(30) Priority: 05.07.2022 JP 2022108556
(71) Applicant: DAIKIN INDUSTRIES, LTD., Osaka-shi, Osaka 530-0001 (JP)
(72) Inventor: SHIRAI, Atsushi, Osaka-Shi, Osaka 530-0001 (JP); ADACHI, Kenji, Osaka-Shi, Osaka 530-0001 (JP); KUROKI, Yoshichika, Osaka-Shi, Osaka 530-0001 (JP); KISHIKAWA, Yosuke, Osaka-Shi, Osaka 530-0001 (JP); NAMIKAWA, Takashi, Osaka-Shi, Osaka 530-0001 (JP)
(74) Representative: Hoffmann Eitle
(86) International application number: PCT/JP2023/024768
(87) International publication number: WO 2024/009998

(57) **Abstract**

Provided is a method for producing a fluorinated organic compound by fluorinating an organic compound in the presence of a fluorinating composition comprising IF₅, iodine, and an amine, the composition comprising the iodine in an amount of 0.12 mol or less per mole of the IF₅. This production method can synthesize a fluorinated organic compound with high efficiency.

## Description

### Technical Field

The present disclosure relates to a method for producing a fluorinated organic compound.

### Background Art

Fluorine compounds are extremely important compounds as various chemical products, such as functional materials, pharmaceutical and agrochemical compounds, and electronic materials, and as intermediates thereof and others.

In recent years, a reaction for introducing a fluorine atom into an organic compound by using nucleophilic substitution with a fluoride ion, and various fluorinating agents for use in the reaction, have been developed.

Iodine pentafluoride (IF₅) is known as a strong fluorinating agent with high oxidizing power; however, iodine pentafluoride is a liquid fluorinating agent that reacts with moisture in air and decomposes while generating HF.

With respect to IF₅ having this property, for example, Patent Literature (PTL) 1 reports in Example 27 that the reaction was allowed to proceed in the presence of an excess amount of iodine (2 equivalents relative to the substrate) as a result of mixing triethylamine and HF to form an IF₅-Et₃N-HF complex.

### Citation List

### Patent Literature

PTL 1: WO01/096263

### Summary of Invention

### Technical Problem

An object of the present disclosure is to provide, for example, a production method capable of efficiently synthesizing a fluorinated organic compound.

### Solution to Problem

The present disclosure includes the following features.
Item 1. A method for producing a fluorinated organic compound, comprising fluorinating an organic compound in the presence of a fluorinating composition, the fluorinating composition comprising IF₅, iodine, and an amine, the fluorinating composition comprising the iodine in an amount of 0.12 mol or less per mole of the IF₅.
Item 2. The method according to Item 1, wherein the content of the iodine is 0.11 mol or less per mole of the IF₅.
Item 3. The method according to Item 1 or 2, wherein the amine comprises at least one amine selected from the group consisting of an aliphatic amine, an alicyclic amine, an aromatic amine, a heterocyclic amine, and a polymer-supported amine.
Item 4. The method according to any one of Items 1 to 3, wherein the amine comprises an aliphatic amine.
Item 5. The method according to Item 3 or 4, wherein the aliphatic amine comprises an aliphatic tertiary amine.
Item 6. The method according to Item 5, wherein the aliphatic tertiary amine comprises triethylamine.
Item 7. The method according to any one of Items 1 to 6, wherein the fluorinating composition further comprises an acid.
Item 8. The method according to Item 7, wherein the acid comprises a Brønsted acid and/or a Lewis acid.
Item 9. The method according to Item 7 or 8, wherein the acid comprises HF.
Item 10. A composition comprising IF₅, iodine, and an amine, the composition comprising the iodine in an amount of 0.12 mol or less per mole of the IF₅.
Item 11. The composition according to Item 10, wherein the content of the iodine is 0.11 mol or less per mole of the IF₅.
Item 12. The composition according to Item 10 or 11, wherein the amine comprises at least one amine selected from the group consisting of an aliphatic amine, an alicyclic amine, an aromatic amine, a heterocyclic amine, and a polymer-supported amine.
Item 13. The composition according to any one of Items 10 to 12, wherein the amine comprises an aliphatic amine.
Item 14. The composition according to Item 12 or 13, wherein the aliphatic amine comprises an aliphatic tertiary amine.
Item 15. The composition according to Item 14, wherein the aliphatic tertiary amine comprises triethylamine.
Item 16. The composition according to any one of Items 10 to 15, further comprising an acid.
Item 17. The composition according to Item 16, wherein the acid comprises a Brønsted acid and/or a Lewis acid.
Item 18. The composition according to Item 16 or 17, wherein the acid comprises HF.
Item 19. A reagent for fluorinating an organic compound, the reagent comprising IF₅, iodine, and an amine, the reagent comprising the iodine in an amount of 0.12 mol or less per mole of the IF₅.

### Effect of the Invention

According to the present disclosure, a production method capable of efficiently synthesizing a fluorinated organic compound can be provided.

### Description of Embodiments

The details and embodiments of the production method, composition, and the like according to the present disclosure are described below. However, it should be understood that various modifications can be made in terms of the details and embodiments without departing from the spirit and scope of the claims.

### 1. Terminology

The symbols and abbreviations in the present specification can be understood in the sense commonly used in the technical field to which the present disclosure pertains, according to the context of the present specification, unless otherwise specified.

In the present specification, the term "comprise" is a concept including "comprising," "consisting essentially of," and "consisting of."

In the present specification, the numerical range indicated by "A to B" means A or more and B or less, unless otherwise specified.

In the present specification, when a group is indicated by a "C_{X}-C_{Y} Z group," it means that the group Z contains X to Y carbon atoms, unless otherwise specified.

The steps, treatments, or operations described in the present specification can be performed at room temperature, unless otherwise specified.

In the present specification, "room temperature" refers to a temperature within the range of 10 to 40°C, and more preferably 15 to 30°C.

In the present disclosure, the term "yield" means a ratio (mol %) of the total molar amount of the target compound contained in outflow gas from the reactor outlet to the molar amount of the starting material compound supplied to the reactor.

### 2. Production Method

The production method according to the present disclosure is a method for producing a fluorinated organic compound, the method comprising the step of fluorinating an organic compound in the presence of a fluorinating composition comprising IF₅, iodine, and an amine, the fluorinating composition containing the iodine in an amount of 0.12 mol or less per mole of IF₅.

According to the present disclosure, a fluorinated organic compound can be efficiently synthesized by using this production method. In particular, in a fluorination reaction using IF₅, the reaction often does not proceed for a certain period at the initial stage of the reaction (an induction period) (e.g., 0 to 30 minutes, particularly 0 to 10 minutes from the start of the reaction). From the viewpoint of stable production, controlling this period is preferable in mass production. According to the present disclosure, the reaction can proceed efficiently even at the initial stage of the reaction (e.g., 0 to 30 minutes, particularly 0 to 10 minutes from the start of the reaction).

### (2-1) Organic Compound (Reaction Substrate)

In the present disclosure, the organic compound that is a reaction substrate is preferably a compound that is fluorinated by a reaction of introducing a fluorine atom into an organic compound, which utilizes a nucleophilic substitution reaction with fluoride ions, and is preferably a hydrogen-atom-containing organic compound that has at least one hydrogen atom.

From the standpoints of facilitating the fluorination reaction and improving efficiency at the initial stage of the reaction, examples of such organic compounds include organic compounds having one or two or more groups, such as hydroxyl, (thio)carbonyl, (thio)ether, imino, (thio)ester, (thio)amide, epoxy, amino, aromatic, alkenyl, alkynyl, and like groups.

In the present disclosure, the fluorination of an organic compound that is a reaction substrate means not only the replacement of hydrogen atoms with fluorine atoms, but also the replacement of the following atoms, groups, etc., particularly the above-mentioned groups or sites adjacent to the above-mentioned groups with fluorine atoms, as will be described later.

Hydrogen atom (CH→CF)

Hydroxyl group (COH→CF)

Carbonyl group (C=O→CF₂)

Thiocarbonyl group (C=S→CF₂)

Sulfide group (CS→CF)

Imino group (C=NH→CF₂)

Hydrazino group (C-NHNH₂→C-F; C=N-NH₂→CF(N=NH)→CF₂)

Epoxy group (C₂O→CF-COH).

Examples of such organic compounds include:
(2A) compounds having a hydroxyl group,
(2B) ketone compounds (including diketones, β-ketocarboxylic acids, β-ketoesters, cyclic ketones, ketals, etc.); aldehyde compounds (including acetals); imine compounds, such as Schiff bases and hydrazones; or ester compounds;
(2C) sulfide compounds,
(2D) epoxy compounds,
(2E) aromatic compounds (e.g., phenylhydrazine derivatives, phenol derivatives, 2-naphthol derivatives, and aniline derivatives),
(2F) thiocarbonyl compounds,
(2G) polyfluorination of the ethyl moiety of -COOR group-containing ethyl sulfide compounds, and
(2H) unsaturated carbon compounds (e.g., olefin compounds).

Among these, from the standpoints of, for example, the reaction conversion rate, selectivity of the target product, yield, and efficiency at the initial stage of the reaction, "(2B) ketone compounds (including diketones, β-ketocarboxylic acids, β-ketoesters, cyclic ketones, ketals, etc.); aldehyde compounds (including acetals); imine compounds such as Schiff bases and hydrazones; or ester compounds," "(2F) thiocarbonyl compounds," and the like are preferred. Among these, ketone compounds (including diketones, β-ketocarboxylic acids, β-ketoesters, cyclic ketones, ketals, etc.); ester compounds; thiocarbonyl compounds, and the like are more preferred. Ketone compounds (including diketones, β-ketocarboxylic acids, β-ketoesters, cyclic ketones, ketals, etc.); thiocarbonyl compounds, and the like are even more preferred. β-Ketoesters, thioketones, dithiocarbamates, and the like are particularly preferred.

Examples of fluorination in the production method according to the present disclosure are described below. The examples of fluorination also show examples of fluorinated organic compounds, i.e., target products obtained by the production method of the present disclosure, as well as examples of organic compounds that are reaction substrates.

From the standpoints of, for example, the reaction conversion rate, selectivity of the target product, yield, and efficiency at the initial stage of the reaction, the target fluorinated organic compound is preferably a compound having at least one partial structure generated by the fluorination: -CF-; more preferably a compound having at least one partial structure generated by the fluorination: -CHF- or -CF₂-; and even more preferably a compound having at least one partial structure generated by the fluorination: -CF₃.

### Fluorination Reaction 1: (2A) Fluorination of Compounds Having a Hydroxyl Group

In this fluorination, for example, the following reaction is performed.

(a) R¹-OH → R¹-F

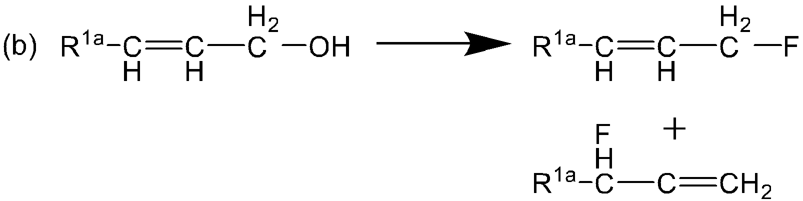

wherein R¹ represents an alkyl group optionally having at least one substituent, an aralkyl group optionally having at least one substituent, an alkenyl group optionally having at least one substituent, an acyl group, a cycloalkyl group optionally having at least one substituent, or a heterocycloalkyl group optionally having at least one substituent; and
R^{1a} represents an alkyl group optionally having at least one substituent, an aryl group optionally having at least one substituent, an aralkyl group optionally having at least one substituent, an alkenyl group optionally having at least one substituent, an acyl group, a cycloalkyl group optionally having at least one substituent, or a heterocycloalkyl group optionally having at least one substituent.

In fluorination reaction 1, examples of the alkyl groups represented by R¹ and R^{1a} include linear or branched C₁-C₁₈ alkyl groups, such as methyl, ethyl, n-propyl, isopropyl, n-butyl, isobutyl, sec-butyl, tert-butyl, n-pentyl, neopentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, and n-octadecyl. From the standpoints of, for example, the reaction conversion rate, selectivity of the target product, yield, and efficiency at the initial stage of the reaction, linear or branched C₁-C₆ alkyl groups, such as methyl, ethyl, n-propyl, isopropyl, n-butyl, isobutyl, sec-butyl, tert-butyl, pentyl, neopentyl, and n-hexyl, are preferred.

In fluorination reaction 1, examples of the aralkyl groups represented by R¹ and R^{1a} include C₇-C₁₀ aralkyl groups, such as 2-phenylethyl, benzyl, 1-phenylethyl, 3-phenylpropyl, and 4-phenylbutyl.

In fluorination reaction 1, examples of the alkenyl groups represented by R¹ and R^{1a} include C₂-C₆ alkenyl groups, such as vinyl, allyl, and 3-butenyl.

In fluorination reaction 1, examples of the acyl groups represented by R¹ and R^{1a} include formyl; linear or branched C₂-C₆ alkanoyl groups, such as acetyl, propionyl, n-butyryl, isobutyryl, valeryl, isovaleryl, and pivaloyl; and C₇-C₁₅ arylcarbonyl groups, such as benzoyl.

Examples of acyl groups include substituted acetyl groups such as chloroacetyl, bromoacetyl, dichloroacetyl, and trifluoroacetyl; alkoxy-substituted acetyl groups such as methoxyacetyl and ethoxyacetyl; alkylthio-substituted acetyl groups such as methylthioacetyl; and substituted benzoyl groups such as phenoxyacetyl, phenylthioacetyl, 2-chlorobenzoyl, 3-chlorobenzoyl, 4-chlorobenzoyl, 4-methylbenzoyl, 4-t-butylbenzoyl, 4-methoxybenzoyl, 4-cyanobenzoyl, and 4-nitrobenzoyl.

In fluorination reaction 1, examples of the cycloalkyl groups represented by R¹ and R^{1a} include C₃-C₈ cycloalkyl groups, such as cyclopropyl, cyclobutyl, cyclopentyl, cyclohexyl, cycloheptyl, and cyclooctyl. From the standpoints of, for example, the reaction conversion rate, selectivity of the target product, yield, and efficiency at the initial stage of the reaction, C₃-C₇ cycloalkyl groups are preferred.

In fluorination reaction 1, examples of the heterocycloalkyl groups represented by R¹ and R^{1a} include groups in which one or more carbon atoms that constitute the ring structure of the cycloalkyl groups described above are substituted with, for example, nitrogen, oxygen, or sulfur.

In fluorination reaction 1, examples of the aryl group represented by R^{1a} include phenyl and naphthyl.

In the present specification, "optionally having a substituent" includes both the case where a substituent is contained (i.e., substituted) and the case where a substituent is not contained (i.e., unsubstituted). For example, an alkyl group optionally having a substituent includes an alkyl group (i.e., an unsubstituted alkyl group) and an alkyl group having a substituent (i.e., a substituted alkyl group).

When each group has a substituent, the number of substituents in the alkyl group having at least one substituent, alkenyl group having at least one substituent, or like groups having at least one substituent can be, for example, 1 to 5, and preferably 1 to 3. Examples of substituents include halogen atoms (e.g., fluorine, chlorine, bromine, and iodine), C₁-C₆ alkoxy groups (e.g., methoxy, ethoxy, n-propoxy, isopropoxy, n-butoxy, isobutoxy, sec-butoxy, tert-butoxy, pentyloxy, and hexyloxy), C₁-C₆ alkylthio groups (e.g., -S-(C₁-C₆ alkyl), wherein the C₁-C₆ alkyl group can be, for example, those mentioned above), cyano, nitro, amino, and hydroxyl. Examples of alkyl groups having a halogen atom include an alkyl group in which a part or all of the hydrogen atoms are substituted with fluorine.

When each group has a substituent, the number of substituents in the aralkyl group having at least one substituent, aryl group having at least one substituent, cycloalkyl group having at least one substituent, heterocycloalkyl group having at least one substituent, or the like can be, for example, 1 to 5, and preferably 1 to 3. Examples of substituents include the above-mentioned C₁-C₆ alkyl groups, halogen atoms (e.g., fluorine, chlorine, bromine, and iodine), C₁-C₆ alkoxy groups (e.g., methoxy, ethoxy, n-propoxy, isopropoxy, n-butoxy, isobutoxy, sec-butoxy, tert-butoxy, pentyloxy, and hexyloxy), and C₁-C₆ alkylthio groups (e.g., -S-(C₁-C₆ alkyl), wherein the C₁-C₆ alkyl group can be, for example, those mentioned above), cyano, nitro, amino, hydroxyl, and like groups.

Specific examples of organic compounds having a hydroxyl group that satisfy the above conditions include:
aliphatic alcohols such as methanol, ethanol, n-propanol, isopropanol, n-butanol, isobutanol, tert-butanol, pentanol, hexanol, octanol, decanol, palmityl alcohol, stearyl alcohol, and oleyl alcohol;
alicyclic alcohols such as benzyl alcohol, mono-, di-, or trisaccharides having at least one unprotected hydroxyl group, cyclohexyl alcohol, and ascorbic acid;
steroid compounds such as steroid alcohols (e.g., cholesterol, cholic acid, and cortisone);
aliphatic monocarboxylic acids such as acetic acid, trifluoroacetic acid, propionic acid, acrylic acid, methacrylic acid, crotonic acid, butyric acid, valeric acid, isovaleric acid, pivalic acid, lauric acid, myristic acid, palmitic acid, stearic acid, oleic acid, linoleic acid, linolenic acid, and cinnamic acid;
polycarboxylic acids, such as oxalic acid, succinic acid, malonic acid, glutaric acid, adipic acid, maleic acid, fumaric acid, and citric acid;
aromatic carboxylic acids, such as benzoic acid, salicylic acid, (o-, m-, p-)phthalic acid, nalidixic acid, and nicotinic acid;
vitamin compounds having a carboxylic acid group, such as pantothenic acid and biotin;
20 kinds of natural amino acids, such as glycine, alanine, phenylalanine, cysteine, aspartic acid, glutamic acid, threonine, histidine, lysine, methionine, and proline; and
carboxylic acid compounds, such as hydroxycarboxylic acids (e.g., lactic acid, citric acid, malic acid, and tartaric acid).

### Fluorination Reaction 2: (2B) Fluorination of Ketone Compounds (Including Diketones, β-Ketocarboxylic Acids, β-Ketoesters, Cyclic Ketones, Ketals, etc.); Aldehyde Compounds (Including Acetals); Schiff Bases, Imine Compounds Such as Hydrazones; or Ester Compounds

In the fluorination, for example, the following reactions take place.

(a-1) 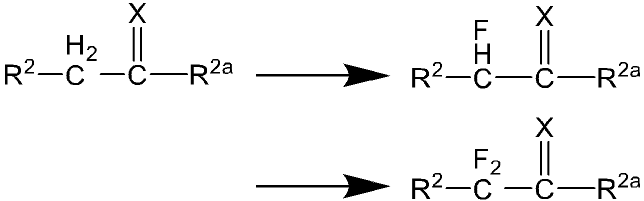
(a-2) 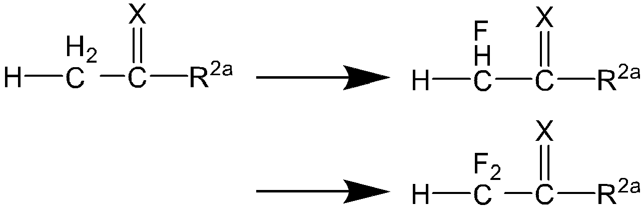
(a-3) 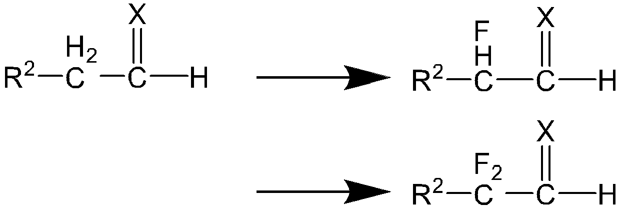
(b-1) 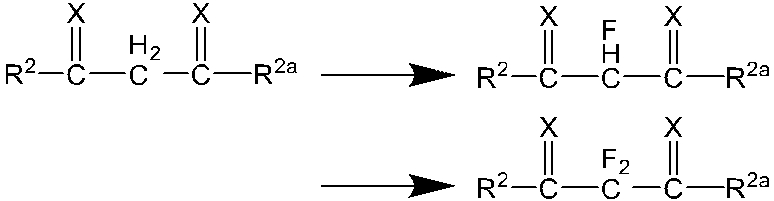
(b-2)

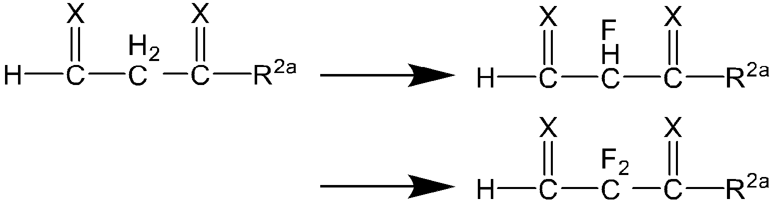

(c) 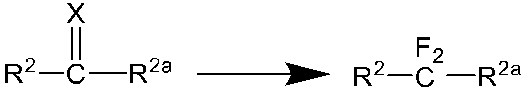
(d) 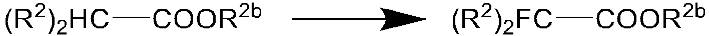
(e-1) 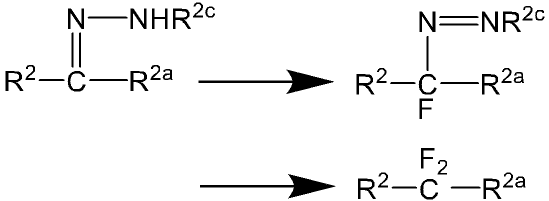
(e-2)

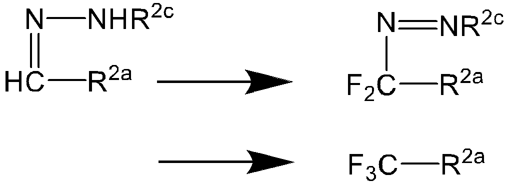

In these formulas, Xs are the same or different, and each represents O or NR' (wherein R' represents a hydrogen atom, an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, a heterocyclic group optionally having at least one substituent, an alkoxy group optionally having at least one substituent, an aryloxy group optionally having at least one substituent, an amino group, a monoalkylamino group optionally having at least one substituent, a dialkylamino group optionally having at least one substituents, an acyl group, or an acylamino group).

R², R^{2a}, and R^{2c} are the same or different, and each represents a hydrogen atom, an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, a heterocyclic group optionally having at least one substituent, an alkoxy group optionally having at least one substituent, an aryloxy group optionally having at least one substituent, a monoalkylamino group optionally having at least one substituent, a dialkylamino group optionally having at least one substituent, an acyl group, or an acylamino group; or R² and R^{2a} may be bonded to each other to form a cyclic structure. R^{2b} represents an alkyl group optionally having at least one substituent, an aralkyl group optionally having at least one substituent, or an aryl group optionally having at least one substituent.

In fluorination reaction 2, examples of the alkyl groups, aralkyl groups, and aryl groups represented by R', R², R^{2a}, R^{2b}, and R^{2c} include those mentioned above. Preferred specific examples, and the types and numbers of substituents are also the same as above.

In fluorination reaction 2, examples of the alkenyl groups, cycloalkyl groups, heterocycloalkyl groups, and acyl groups represented by R', R², R^{2a}, and R^{2c} include those mentioned above. Preferred specific examples, and the types and numbers of substituents are also the same as above.

In fluorination reaction 2, examples of the heterocyclic groups represented by R', R², R^{2a}, and R^{2c} include 5-to 10-membered monocyclic or bicyclic heterocyclic groups having at least one heteroatom selected from nitrogen, oxygen, and sulfur as a ring-constituting atom, such as piperidyl, furyl, thienyl, imidazolyl, oxazolyl, thiazolyl, pyrrolyl, pyrrolidinyl, triazolyl, benzothiazolyl, benzimidazolyl, oxadiazolyl, thiadiazolyl, indolyl, pyrazolyl, pyridazinyl, cinnolinyl, quinolyl, isoquinolyl, quinoxalinyl, pyrazinyl, pyridyl, benzofuryl, benzothienyl, and tetrazolyl.

In the present specification, among the heterocyclic groups, examples of the aromatic heterocyclic group include 5- to 10-membered monocyclic or bicyclic heteroaryl groups having at least one heteroatom selected from the group consisting of nitrogen, oxygen, and sulfur as a ring-constituting atom, such as furyl, thienyl, imidazolyl, oxazolyl, thiazolyl, pyrrolyl, triazolyl, benzothiazolyl, benzimidazolyl, oxadiazolyl, thiadiazolyl, indolyl, pyrazolyl, pyridazinyl, cinnolinyl, quinolyl, isoquinolyl, quinoxalinyl, pyrazinyl, pyridyl, benzofuryl, benzothienyl, and tetrazolyl.

In fluorination reaction 2, examples of the alkoxy groups represented by R', R², R^{2a}, and R^{2c} include linear or branched C₁-C₆ alkoxy groups, such as methoxy, ethoxy, n-propoxy, isopropoxy, n-butoxy, isobutoxy, sec-butoxy, tert-butoxy, pentyloxy, and hexyloxy.

In fluorination reaction 2, examples of the aryloxy groups represented by R', R², R^{2a}, and R^{2c} include phenoxy and naphthyloxy.

In fluorination reaction 2, examples of the monoalkylamino groups represented by R', R², R^{2a}, and R^{2c} include amino groups mono-substituted with the C₁-C₆ alkyl groups described above.

In fluorination reaction 2, examples of the dialkylamino groups represented by R', R², R^{2a}, and R^{2c} include amino groups di-substituted with the C₁-C₆ alkyl groups described above, such as dimethylamino, diethylamino, di-n-propylamino, diisopropylamino, dibutylamino, dipentylamino, and dihexylamino.

In fluorination reaction 2, examples of the acylamino groups represented by R', R², R^{2a}, and R^{2c} include formylamino, benzoylamino, acetylamino, propionylamino, n-butyrylamino, and like C₁-C₈ acylamino groups (e.g., formylamino, alkanoylamino, and arylcarbonylamino groups).

When an alkoxy group has a substituent, the number of substituents in the alkoxy group having at least one substituent can be, for example, 1 to 5, and preferably 1 to 3. Examples of the substituents include halogen atoms (e.g., fluorine, chlorine, bromine, and iodine), C₁-C₆ alkoxy groups (e.g., methoxy, ethoxy, n-propoxy, isopropoxy, n-butoxy, isobutoxy, sec-butoxy, tert-butoxy, pentyloxy, and hexyloxy), C₁-C₆ alkylthio groups (e.g., -S-(C₁-C₆ alkyl); wherein the C₁-C₆ alkyl group can be, for example, those mentioned above), cyano, nitro, amino, hydroxyl, and like groups. Examples of the alkyl groups having a halogen atom include an alkyl group in which a part or all of the hydrogen atoms are substituted with fluorine.

When each group has a substituent, the number of substituents in the heterocyclic group having at least one substituent, aryloxy group having at least one substituent, monoalkylamino group having at least one substituent, dialkylamino group having at least one substituent, acylamino group having at least one substituent, and the like can be, for example, 1 to 5, and preferably 1 to 3. Examples of the substituents include the above-mentioned C₁-C₆ alkyl groups, halogen atoms (e.g., fluorine, chlorine, bromine, and iodine), C₁-C₆ alkoxy groups (e.g., methoxy, ethoxy, n-propoxy, isopropoxy, n-butoxy, isobutoxy, sec-butoxy, tert-butoxy, pentyloxy, and hexyloxy), and C₁-C₆ alkylthio groups (e.g., -S-(C₁-C₆ alkyl), wherein the C₁-C₆ alkyl group can be, for example, those mentioned above), cyano, nitro, amino, hydroxyl, and like groups.

Examples of the cyclic structure that may be formed by bonding of R² and R^{2a} to each other include aliphatic 4- to 12-membered rings optionally having at least one substituent. Preferred are 4- to 7-membered aliphatic rings.

Examples of aliphatic 4- to 7-membered aliphatic rings as cyclic structures that may be formed by bonding of R² to R^{2a} to each other include C₄-C₇ cycloalkane rings, such as a cyclobutane ring, a cyclopentane ring, a cyclohexane ring, and a cycloheptane ring.

When each group has a substituent, the number of substituents in the 4- to 7-membered aliphatic ring having at least one substituent can be, for example, 1 to 5, and preferably 1 to 3. Examples of the substituents include the above-mentioned C₁-C₆ alkyl groups, halogen atoms (e.g., fluorine, chlorine, bromine, and iodine), C₁-C₆ alkoxy groups (e.g., methoxy, ethoxy, n-propoxy, isopropoxy, n-butoxy, isobutoxy, sec-butoxy, tert-butoxy, pentyloxy, and hexyloxy), and C₁-C₆ alkylthio groups (e.g., -S-(C₁-C₆ alkyl); wherein the C₁-C₆ alkyl group can be, for example, those mentioned above), cyano, nitro, amino, hydroxyl, and carboxyl ester groups (alkoxy-CO- groups; wherein the alkoxy group can be, for example, those mentioned above). Further, the structure represented by 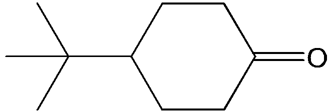 is also included within the scope of the 4- to 7-membered aliphatic ring having at least one substituent.

Examples of the ketone compounds that satisfy the above conditions include acetone, methyl ethyl ketone, acetylacetone, acetoacetic acid, acetoacetic acid esters (e.g., methyl acetoacetate and ethyl acetoacetate), cyclohexanone, acetophenone, benzophenone, propiophenone, 4-piperidone, 1-oxo-1,2-dihydronaphthalene, benzylideneacetophenone (chalcone), and deoxobenzoin, as well as ketals of these compounds.

Examples of the aldehyde compounds that satisfy the above conditions include acetaldehyde, propionaldehyde, butylaldehyde, isobutylaldehyde, valeraldehyde, isovaleraldehyde, acrylaldehyde, benzaldehyde, cinnamaldehyde, anisaldehyde, and nicotinaldehyde, as well as acetals of these compounds.

Examples of the imine compounds, such as Schiff bases and hydrazones, include condensates of ketone compounds or aldehyde compounds, such as those mentioned above, with an appropriate primary amine (e.g., methylamine, ethylamine, propylamine, butylamine, pentylamine, hexylamine, cyclohexylamine, or ethylenediamine) or hydrazine.

Examples of the ester compounds that satisfy the above conditions include methyl isobutyrate and ethyl isobutyrate.

### Fluorination Reaction 3: (2C) Fluorination of Sulfide Compounds (Including Dithioacetal, Dithioketal, etc.)

In this fluorination, for example, a reaction is carried out in which one or two hydrogen atoms of a methylene adjacent to the sulfur atom are substituted with fluorine atoms, or the sulfur atom is substituted with fluorine.

(a-1) 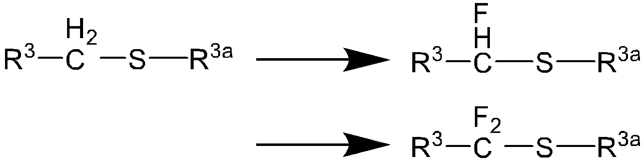
(a-2) 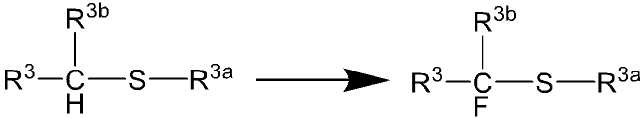
(b-1) 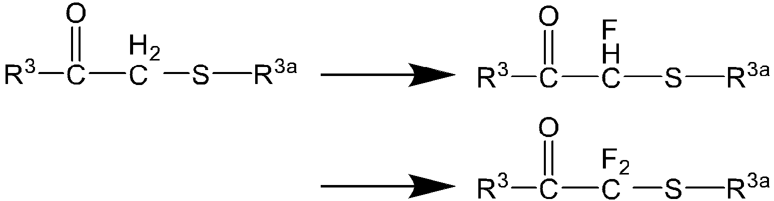
(b-2)

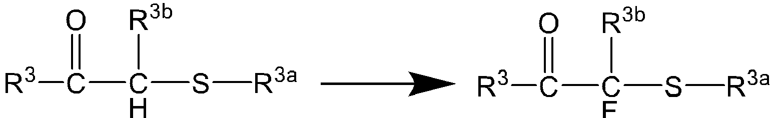

(c) 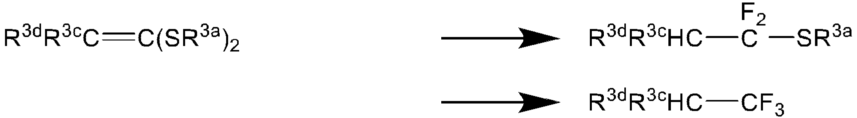
(d) 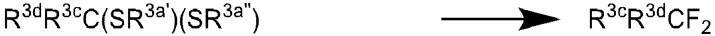
(e) 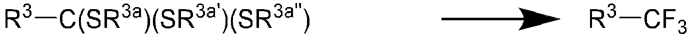
(f) 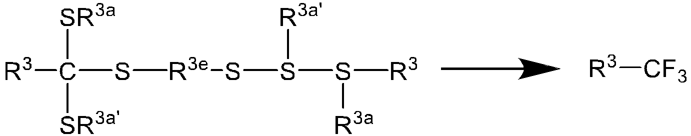
wherein
R^{3a}, R^{3a'} and R^{3a"} are the same or different, and each represents an alkyl group optionally having at least one substituent, an aralkyl group optionally having at least one substituent, an aryl optionally having at least one substituent, an alkenyl group optionally having at least one substituent, a cycloalkyl group optionally having at least one substituent, a heterocycloalkyl group optionally having at least one substituent, or a heterocyclic group optionally having at least one substituent; or
R^{3a} and R^{3a'} taken together represent a 4- to 7-membered aliphatic ring optionally having at least one substituent.
R³ and R^{3b} are the same or different, and each represents an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl optionally having at least one substituent, a heterocyclic group optionally having at least one substituent, an alkoxy group optionally having at least one substituent, an aryloxy group optionally having at least one substituent, an amino group, a monoalkylamino optionally having at least one substituent, a dialkylamino group optionally having at least one substituent, an acyl group, an acylamino group, a cyano group, an alkylsulfinyl group optionally having at least one substituent, an arylsulfinyl group optionally having at least one substituent, a cycloalkylsulfinyl group optionally having at least one substituent, a heterocycloalkylsulfinyl group optionally having at least one substituent, a sulfonyl group to which a heterocyclic group optionally having at least one substituent is bonded, an alkylsulfonyl group optionally having at least one substituent, an aralkylsulfonyl group optionally at least one substituent, an arylsulfonyl group optionally having at least one substituent, a cycloalkylsulfonyl group optionally having at least one substituent, a heterocycloalkylsulfonyl group optionally having at least one substituent, or a sulfonyl group to which a heterocyclic group optionally having at least one substituent is bonded. Alternately, R³ and R^{3b}, taken together with the carbon atom to which they are bonded, optionally form a 4- to 8-membered ring via or not via a heteroatom (the ring is optionally substituted with at least one substituent selected from the group consisting of halogen atoms, oxo groups, alkyl groups optionally having at least one substituent, aralkyl groups optionally having at least one substituent, aryl groups optionally having at least one substituent, alkenyl groups optionally having at least one substituent, cyano groups, and amino groups).
R^{3c} and R^{3d} may be the same or different, and each represents a hydrogen atom, an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, a heterocyclic group optionally having at least one substituent, an alkoxy group optionally having at least one substituent, an aryloxy group optionally having at least one substituent, a monoalkylamino group optionally having at least one substituent, a dialkylamino group optionally having at least one substituent, an acyl group, or an acylamino group. Alternately, R^{3c} and R^{3d}, taken together with the carbon atom adjacent thereto, optionally form a saturated or unsaturated 4-to 7-membered aliphatic ring optionally having at least one substituent (this ring may be substituted with at least one member selected from the group consisting of halogen atoms, oxo groups, alkyl groups optionally having at least one substituent, aralkyl groups optionally having at least one substituent, aryl groups optionally having at least one substituent, alkenyl groups optionally having at least one substituent, cyano groups, and amino groups, and
R^{3e} represents an alkylene group or an arylene group).

In fluorination reaction 3, examples of the alkyl groups, aralkyl groups, aryl groups, alkenyl groups, cycloalkyl groups, heterocycloalkyl groups, and heterocyclic groups represented by R³, R^{3a}, R^{3b}, R^{3c}, R^{3d}, R^{3a'} and R^{3a"} include those described above. Preferred specific examples, and the types and numbers of substituents are also the same as above.

In fluorination reaction 3, examples of the alkoxy groups, aryloxy groups, monoalkylamino groups, dialkylamino groups, acyl groups, and acylamino groups represented by R^{3d} and R^{3d} include those mentioned above. Preferred specific examples, and the types and numbers of substituents are also the same as above.

In fluorination reaction 3, examples of the 4- to 7-membered aliphatic ring that can be formed by R^{3a} and R^{3a'}, and R^{3c} and R^{3d} together include those mentioned above. Preferred specific examples, and the types and numbers of substituents are also the same as above.

In fluorination reaction 3, examples of the alkyl group, aralkyl group, aryl group, cycloalkyl group, heterocycloalkyl group, and heterocyclic group of the alkylsulfinyl group optionally having at least one substituent, aralkylsulfinyl group optionally having at least one substituent, arylsulfinyl group optionally having at least one substituent, cycloalkylsulfinyl group optionally having at least one substituent, heterocycloalkylsulfinyl group optionally having at least one substituent, and sulfinyl group to which a heterocyclic group is bonded, which are represented by R', R², R^{2a}, and R^{2c}, include those mentioned above. Preferred specific examples, and the types and numbers of substituents are also the same as above.

In fluorination reaction 3, examples of the alkyl group, aralkyl group, aryl group, cycloalkyl group, heterocycloalkyl group, and heterocyclic group of the alkylsulfonyl group optionally having at least one substituent, aralkylsulfonyl group optionally having at least one substituent, arylsulfonyl group optionally having at least one substituent, cycloalkylsulfonyl group optionally having at least one substituent, heterocycloalkylsulfonyl group optionally having at least one substituent, and sulfonyl group to which a heterocyclic group is bonded, which are represented by R', R², R^{2a}, and R^{2c}, include those mentioned above. Preferred specific examples, and the types and numbers of substituents are also the same as above.

In fluorination reaction 3, when each group has a substituent, the number of substituents of the alkylsulfinyl group, aralkylsulfinyl group, arylsulfinyl group, cycloalkylsulfinyl group, heterocycloalkylsulfinyl group, sulfinyl group to which a heterocyclic group is bonded, alkylsulfonyl group, aralkylsulfonyl group, arylsulfonyl group, cycloalkylsulfonyl group, heterocycloalkylsulfonyl group, and sulfonyl group to which a heterocyclic group is bonded, which are represented by R', R², R^{2a}, and R^{2c}, can be, for example, 1 to 5, and preferably 1 to 3. Examples of substituents include the above-mentioned C₁-C₆ alkyl groups, halogen atoms (e.g., fluorine, chlorine, bromine, and iodine), C₁-C₆ alkoxy groups (e.g., methoxy, ethoxy, n-propoxy, isopropoxy, n-butoxy, isobutoxy, sec-butoxy, tert-butoxy, pentyloxy, and hexyloxy), and C₁-C₆ alkylthio groups (e.g., -S-(C₁-C₆ alkyl); wherein the C₁-C₆ alkyl group can be, for example, those mentioned above), cyano, nitro, amino, hydroxyl, and carboxyl ester groups (e.g., alkoxy-CO-, wherein the alkoxy group can be, for example, those mentioned above).

In fluorination reaction 3, examples of the 4- to 8-membered ring that can be formed by bonding of R³ and R^{3b} to each other together with the carbon atom to which they are bonded via or not via a heteroatom include a cyclobutane ring, a cyclopentane ring, a cyclohexane ring, a cycloheptane ring, a cyclooctane ring, a piperidine ring, a furan ring, a thiophene ring, an imidazole ring, an oxazole ring, a thiazole ring, a pyrrole ring, a pyrrolidine ring, and a triazine ring.

In fluorination reaction 3, when the 4- to 8-membered ring that can be formed by bonding R³ and R^{3b} together with the carbon atom to which they are bonded via or not via a heteroatom has at least one substituent, the number of substituents may have 1 to 5, and preferably 1 to 3. Examples of substituents include halogen atoms (e.g., fluorine, chlorine, bromine, and iodine), oxo, the above-mentioned alkyl groups, the above-mentioned alkenyl groups, cyano, and amino.

In fluorination reaction 3, examples of the alkylene group represented by R^{3e} include linear or branched C₁-C₁₈ alkylene groups, such as methylene, ethylene, trimethylene, propylene, and tetramethylene, and preferably linear or branched C₁-C₆ alkylene groups.

In fluorination reaction 3, examples of the arylene group represented by R^{3e} include a phenylene group (e.g., 1,4-phenylene) and a naphthylene group (e.g., 2,6-naphthylene).

Examples of the sulfide compounds that satisfy the above conditions include methyl ethyl sulfide, methyl benzyl sulfide, 2-phenylthioacetic acid ester, 2-phenylthioacetophenone, 2-(methylthio)acetophenone, bis(methylthio)methylbenzene, 2-octyl-1,3-dithiane, 2-phenyl-2-trifluoromethyl-1,3-dithiolane, tris(ethylthio)hexane, and 4-tris(methylthio)toluene.

### Fluorination Reaction 4: (2D) Fluorination of Epoxy Compound

In this fluorination, a fluorine addition reaction is carried out, for example, according to the following reaction: 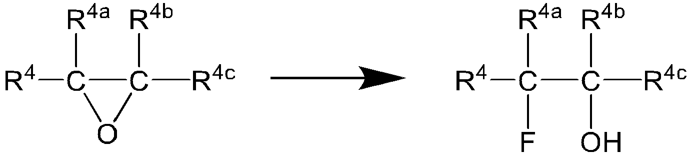 wherein R⁴, R^{4a}, R^{4b}, and R^{4C} are the same or different, and each represents a hydrogen atom, an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, or a heterocyclic group optionally having at least one substituent.

In fluorination reaction 4, examples of the alkyl groups, aralkyl groups, aryl groups, alkenyl groups, cycloalkyl groups, heterocycloalkyl groups, and heterocyclic groups represented by R⁴, R^{4a}, R^{4b} and R^{4c} include those mentioned above. Preferable specific examples, and the types and numbers of substituents can be the same as above.

Examples of epoxy compounds that satisfy the above conditions include oxirane, 1,2-epoxyethylbenzene, 1-chloro-2,3-epoxypropane, and α,α'-epoxybibenzyl.

### Fluorination Reaction 5: (2E) Fluorination of Aromatic Compounds

In the fluorination, a fluorine substituent is introduced into an aromatic ring, for example, by the following reaction. Fluorination of the aromatic ring of the phenol derivative or aniline derivative can be carried out by fluorination followed by reduction with a reducing agent, such as zinc powder, to give the desired fluoride.

### Fluorination Reaction 5-1: Fluorination of Phenylhydrazine Derivative

The phenylhydrazine residue optionally having at least one substituent can be substituted with a fluorine atom.

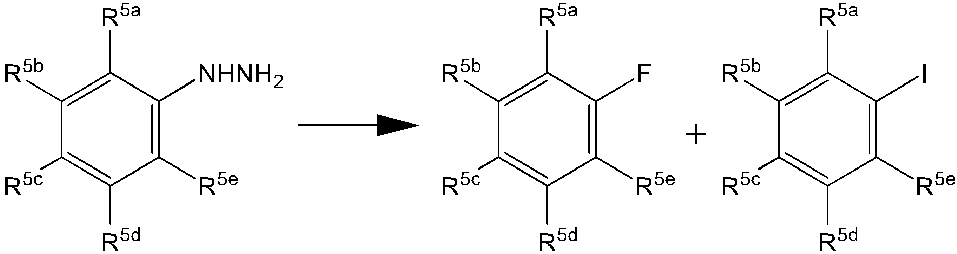

In the formula, R^{5a}, R^{5b}, R^{5c}, R^{5d} and R^{5e} are the same or different, and each represents a hydrogen atom, an alkyl group optionally having at least one substituent, an aralkyl group optionally having at least one substituent, an aryl group optionally having at least one substituent, an alkoxy group optionally having at least one substituent, a nitro group, a cyano group, a halogen atom, an acyl group, an arylcarbonyl group optionally having at least one substituent, an amino group, a monoalkylamino group optionally having at least one substituent, a dialkylamino group optionally having at least one substituent, an acylamino group optionally having at least one substituent, an arylcarbonylamino group optionally having at least one substituent, or an alkylthio group optionally having at least one substituent.

In fluorination reaction 5-1, examples of the alkyl groups, aralkyl groups, aryl groups, alkoxy groups, acyl groups, monoalkylamino groups, dialkylamino groups, and acylamino groups represented by R^{5a}, R^{5b}, R^{5c}, R^{5d}, and R^{5e} include those mentioned above. Preferred specific examples, and the types and numbers of substituents are also the same as above.

In fluorination reaction 5-1, examples of the halogen atoms represented by R^{5a}, R^{5b}, R^{5c}, R^{5d}, and R^{5e} include fluorine, chlorine, bromine, and iodine.

In fluorination reaction 5-1, examples of the aryl group of the arylcarbonyl groups represented by R^{5a}, R^{5b}, R^{5c}, R^{5d}, and R^{5e} include those mentioned above. Preferred specific examples, and the types and numbers of substituents are also the same as above.

In fluorination reaction 5-1, examples of the arylcarbonyl group of the arylcarbonylamino groups represented by R^{5a}, R^{5b}, R^{5c}, R^{5d}, and R^{5e} include those mentioned above. Preferred specific examples, and the types and numbers of substituents are also the same as above.

In fluorination reaction 5-1, examples of the alkylthio groups represented by R^{5a}, R^{5b}, R^{5c}, R^{5d}, and R^{5e} include -S-(C₁-C₆ alkyl) and like groups. The C₁-C₆ alkyl groups can be, for example, those mentioned above. Preferred specific examples, and the types and numbers of substituents are also the same as above.

### Fluorination Reaction 5-2: Fluorination of Phenol Derivative

When reacted with IF₅, phenol derivatives form difluorinated quinonoid structures as shown below, and subsequent reduction of the resulting compounds produces phenol derivatives having fluorine introduced in the ortho- or para-position.

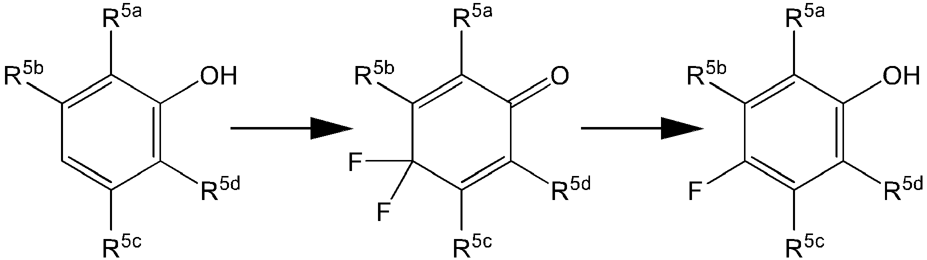

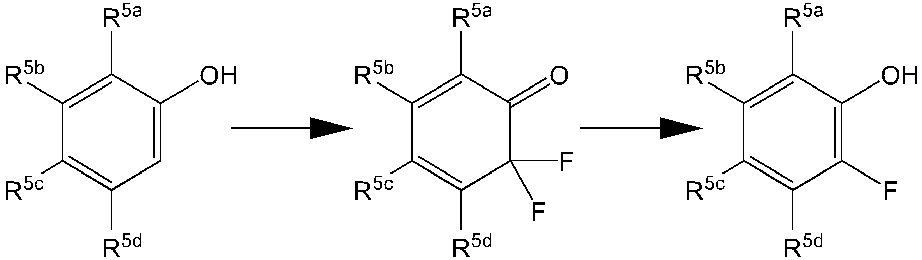

In the formula, R^{5a}, R^{5b}, R^{5c}, and R^{5d} are the same or different, and each represents a hydrogen atom, an alkyl group optionally having at least one substituent, an aralkyl group optionally having at least one substituent, an aryl group optionally having at least one substituent, an alkoxy group optionally having at least one substituent, a nitro group, a cyano group, a halogen atom, an acyl group, an arylcarbonyl group optionally having at least one substituent, an amino group, a monoalkylamino optionally having at least one substituent, a dialkylamino group optionally having at least one substituent, an acylamino group optionally having at least one substituent, an arylcarbonylamino group optionally having at least one substituent, or an alkylthio group optionally having a substituent.

In fluorination reaction 5-2, examples of the alkyl groups, aralkyl groups, aryl groups, alkoxy groups, halogen atoms, acyl groups, arylcarbonyl groups, monoalkylamino groups, dialkylamino groups, acylamino groups, arylcarbonylamino groups, and alkylthio groups represented by R^{5a}, R^{5b}, R^{5c}, R^{5d}, and R^{5e} include those described above. Preferred specific examples, and the types and numbers of substituents are also the same as above.

When a starting material whose ortho- and para-positions have been all substituted is used, a fluorine atom is introduced into the ortho-position or para-position to form a compound having a fluorinated quinonoid structure.

In the above example, phenol optionally having at least one substituent is used as a phenol derivative; however, it is also possible to use a benzene-based aromatic compound or a condensed polycyclic hydrocarbon that has an electron-releasing group, such as a hydroxyl group or an alkoxy group, and that is optionally further substituted, to introduce a fluorine atom thereinto in a similar manner.

### Fluorination Reaction 5-3: Fluorination of 2-Naphthol Derivatives

The 1-position of the naphthol can be mono- or difluorinated.

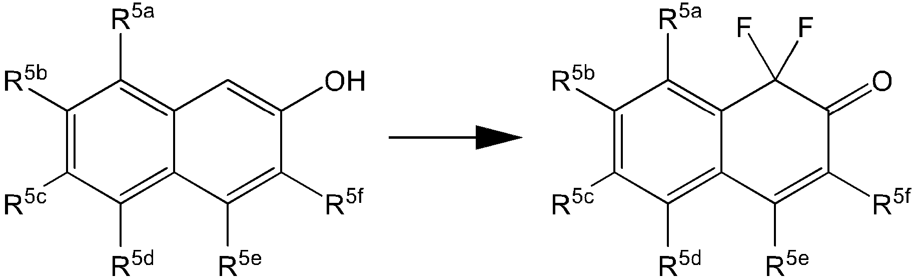

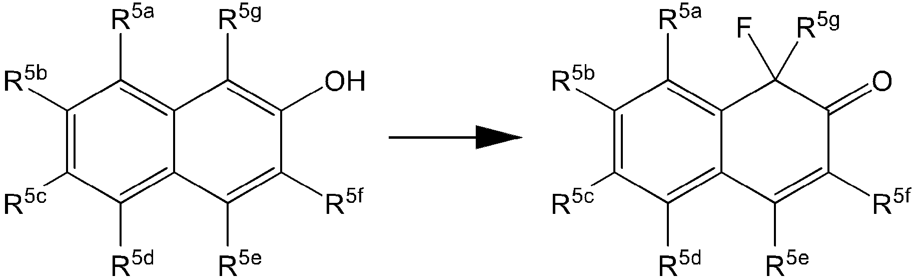

In the formulas, R^{5a}, R^{5b}, R^{5c}, R^{5d}, R^{5e}, R^{5f}, and R^{5g} are the same or different, and each represents a hydrogen atom, an alkyl group optionally having at least one substituent, an aralkyl group optionally having at least one substituent, an aryl group optionally having at least one substituent, an alkoxy group optionally having at least one substituent, a nitro group, a cyano group, a halogen atom, an acyl group, an arylcarbonyl group optionally having at least one substituent, an amino group, a monoalkylamino group, a dialkylamino group, an acylamino group, an arylcarbonylamino, or an alkylthio group optionally having at least one substituent.

In fluorination reaction 5-3, examples of the alkyl group, aralkyl group, aryl group, alkoxy group, halogen atom, acyl group, arylcarbonyl group, monoalkylamino group, dialkylamino group, acylamino group, arylcarbonylamino group, and alkylthio groups represented by R^{5a}, R^{5b}, R^{5c}, R^{5d}, R^{5e}, R^{5f}, and R^{5g} include those described above. Preferred specific examples, and the types and numbers of substituents are also the same as above.

### Fluorination Reaction 5-4: Fluorination of Aniline Derivatives

Like phenol derivatives, aniline derivatives also form difluorinated quinonoid structures when reacted with IF₅ as shown below, and subsequent reduction of the resulting compounds produces aniline derivatives having fluorine introduced in the ortho- or para-position.

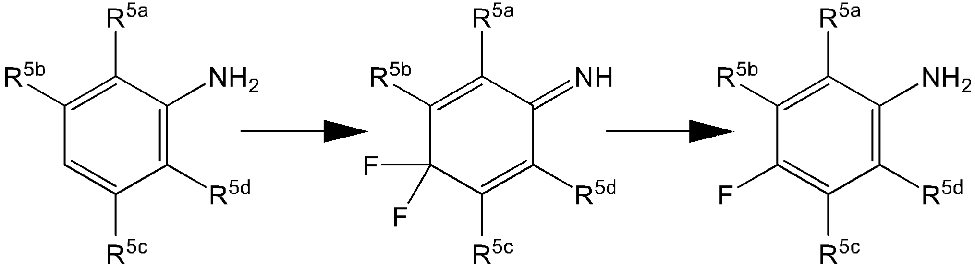

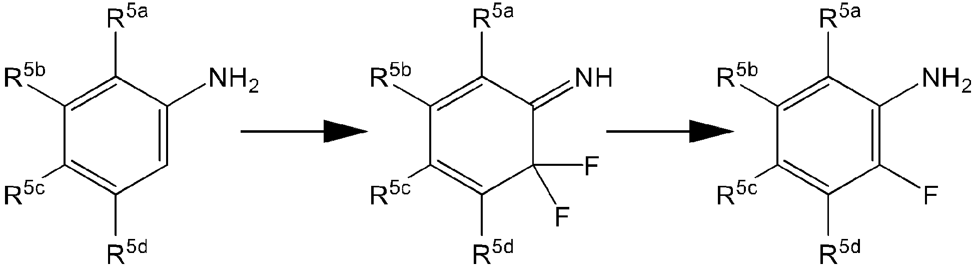

In the formula, R^{5a}, R^{5b}, R^{5c}, and R^{5d} are the same or different, and each represents a hydrogen atom, an alkyl group optionally having at least one substituent, an aralkyl group optionally having at least one substituent, an aryl group optionally having at least one substituent, an alkoxy group optionally having at least one substituent, a nitro group, a cyano group, a halogen atom, an acyl group, an arylcarbonyl optionally having at least one substituent, an amino group, a monoalkylamino group, a dialkylamino group, an acylamino group, an arylcarbonylamino group, or an alkylthio group optionally having at least one substituent.

In fluorination reaction 5-4, examples of the alkyl groups, aralkyl groups, aryl groups, alkoxy groups, halogen atom, acyl groups, arylcarbonyl groups, monoalkylamino groups, dialkylamino groups, acylamino groups, arylcarbonylamino groups, and alkylthio groups represented by R^{5a}, R^{5b}, R^{5c}, R^{5d}, and R^{5e} include those described above. Preferred specific examples, and the types and numbers of substituents are also the same as above.

It is also possible to use an aniline derivative, such as aniline optionally having at least one substituent or naphthylamine optionally having at least one substituent to introduce a fluorine atom into the aromatic ring in a similar manner.

### Fluorination Reaction 6: (2F) Fluorination of Thiocarbonyl Compounds (Including Thioketone, Thioester, Thiocarbonic Ester, Thioamide, Dithiocarboxylate, and Dithiocarbamate)

This reaction is for fluorination of a compound having the partial structure: -C(=S)-Y-, wherein Y is O, S, or a single bond. For example, the following reactions can be performed.

(a) 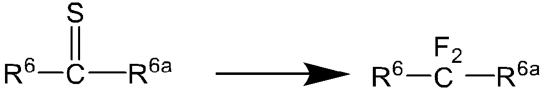
(b)

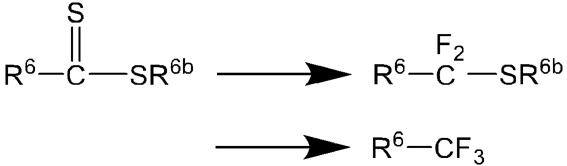

In the formula, R⁶ and R^{6a} are the same or different, and each represents a hydrogen atom, an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, a heterocyclic group optionally having at least one substituent, an alkoxy group optionally having at least one substituent, an aryloxy group optionally having at least one substituent, a monoalkylamino group optionally having at least one substituent, a dialkylamino group optionally having at least one substituent, an acyl group, or an acylamino group;
or R⁶ and R^{6a} may be bonded to each other to form a cyclic structure.
R^{6b} represents an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, or a heterocyclic group optionally having at least one substituent.

In fluorination reaction 6, examples of the alkyl group, aralkyl group, aryl group, alkenyl group, cycloalkyl group, heterocycloalkyl group, and heterocyclic group represented by R⁶, R^{6a}, and R^{6b} include those mentioned above. Preferred specific examples, and the types and numbers of substituents are also the same as above.

In fluorination reaction 6, examples of the alkoxy group, aryloxy group, monoalkylamino group, dialkylamino group, acyl group, and acylamino group represented by R⁶ and R^{6a} include those mentioned above. Preferred specific examples, and the types and numbers of substituents are also the same as above.

In fluorination reaction 6, examples of the cyclic structure that can be formed by R⁶ and R^{6a} together include those described above. Preferred specific examples, and the types and numbers of substituents are also the same as above.

In fluorination reaction 6, when the amount of the fluorinating agent used in the reaction of formula (b) is reduced (e.g., 0.5 equivalents or less), the amount of R⁶-CF₂-SR^{6b} formed becomes dominant.

Examples of the thiocarbonyl compounds that satisfy the above conditions include O-(4-isopropylphenyl) S-methyl dithiocarbonate, O-(4-bromophenyl) S-methyl dithiocarbonate, ethyl 4-(((methylthio)carbonothioyl)oxy)benzoate, O-decyl S-methyl dithiocarbonate, O-(3-phenylpropyl) S-methyl dithiocarbonate, O-methyl cyclohexanecarbothioate, O-propyl 1-piperidinecarbothioate, methyl dithiobenzoate, thiobenzophenone, O-phenyl thiobenzoate, N,N-dimethylphenylthioamide, ethyl 3-quinolinedithiocarboxylate, trifluoromethanecarbothioylnaphthalene, N-methyl-N-phenyltrifluoromethanethicamide, N-benzyl-N-phenylheptafluoropropanethioamide, and O-(4'-pentyl-[1,1'-bi(cyclohexane)]-4-yl)S-methyl dithiocarbonate, 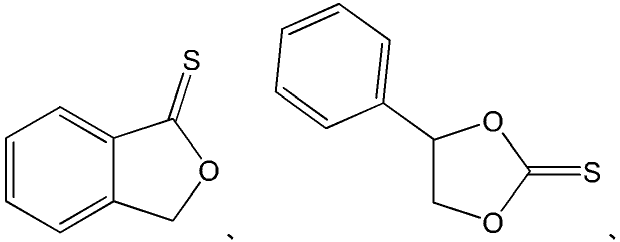 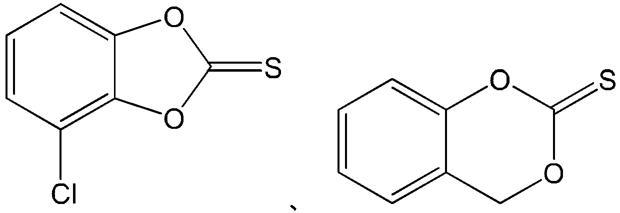 etc.

### Fluorination Reaction 7: (2G) Polyfluorination of Ethyl Moiety of Ethyl Sulfide Compound having -COOR Group

In this fluorination, the ethyl moiety adjacent to the sulfur atom is polyfluorinated.

The reaction proceeds, for example, according to the following reaction scheme:

R⁷-S-CH(COOR^{7a})-CH₃→R⁷-S-CHF-CF₂-COOR^{7a}

In the formula, R⁷ represents an aryl group optionally having at least one substituent or an aromatic heterocyclic group optionally having at least one substituent.
R^{7a} represents a hydrogen atom, an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, a heterocyclic group optionally having at least one substituent, an alkoxy group optionally having at least one substituent, an aryloxy group optionally having at least one substituent, an amino group, a monoalkylamino group optionally having at least one substituent, a dialkylamino group optionally having at least one substituent, an acyl group, an acylamino group, a cyano group, an alkylsulfinyl group optionally having at least one substituent, an aralkylsulfinyl group optionally having at least one substituent, an arylsulfinyl group optionally having at least one substituent, a cycloalkylsulfinyl group optionally having at least one substituent, a heterocycloalkylsulfinyl group optionally having at least one substituent, a sulfinyl group to which a heterocyclic group optionally having at least one substituent is bonded, an alkylsulfonyl group optionally having at least one substituent, an aralkylsulfonyl group optionally having at least one substituent, an arylsulfonyl group optionally having at least one substituent, a cycloalkylsulfonyl group optionally having at least one substituent, a heterocycloalkylsulfonyl group optionally having at least one substituent, or a sulfonyl group to which a heterocyclic group optionally having at least one substituent is bonded.

In fluorination reaction 7, the aryl groups represented by R⁷ and R^{7a} can be those mentioned above. Preferred specific examples, and the types and numbers of substituents are also the same as above.

In fluorination reaction 7, examples of the alkyl group, aralkyl group, alkenyl group, cycloalkyl group, heterocycloalkyl group, heterocyclic group, alkoxy group, aryloxy group, monoalkylamino group, dialkylamino group, acyl group, acylamino group, alkylsulfinyl group, aralkylsulfinyl group, arylsulfinyl group, cycloalkylsulfinyl group, heterocycloalkylsulfinyl group, sulfinyl group to which a heterocyclic group is bonded, alkylsulfonyl group, aralkylsulfonyl group, arylsulfonyl group, cycloalkylsulfonyl group, heterocycloalkylsulfonyl group, and sulfonyl group to which a heterocyclic group is bonded, which are represented by R^{7a}, include those mentioned above. Preferred specific examples, and the types and numbers of substituents are also the same as above.

An example of an ethyl sulfide compound having a -COOR group that satisfies the above conditions is ethyl 2-((4-chlorophenyl)thio)propanoate.

### Fluorination Reaction 8: Fluorination of (2H) Unsaturated Carbon Compound

In this fluorination, fluorine or iodine is added to a carbon-carbon double bond or a carbon-carbon triple bond.

The reaction proceeds, for example, according to the following:

(a) R^{8a}R^{8a'}C=CR^{8b}R^{8b'} → FR^{8a}C-CR^{8b}I

(b) R^{8a}C≡CR^{8b} → FR^{8a}C=CR^{8b}I

In the formulas, R^{8a}, R^{8a'}, R^{8b}, and R^{8b'} are the same or different, and each represents a hydrogen atom, an alkyl group optionally having at least one substituent, an aralkyl group optionally having at least one substituent, an aryl group optionally having at least one substituent, an alkenyl group optionally having at least one substituent, an acyl group, a cycloalkyl group optionally having at least one substituent, a heterocycloalkyl group optionally having at least one substituent, an ester group, or a halogen atom. Alternatively, at least two of the R^{8a}, R^{8a'} , R^{8b}, and R^{8b'} may be bonded to each other to form a ring structure.

In fluorination reaction 8, examples of the alkyl groups, aralkyl groups, aryl groups, alkenyl groups, acyl groups, cycloalkyl groups, heterocycloalkyl groups, and halogen atoms represented by R^{8a}, R^{8a'}, R^{8b}, and R^{8b'} include those mentioned above. Preferred specific examples, and the types and numbers of substituents are also the same as above.

In fluorination reaction 8, examples of the cyclic structure that can be formed by combining two or more of R^{8a}, R^{8a'}, R^{8b}, and R^{8b'} include those mentioned above. Preferred specific examples, and the types and numbers of substituents are also the same as above.

In fluorination reaction 8, examples of the ester groups represented by R^{8a}, R^{8a'}, R^{8b} and R^{8b"} include an acyl-O-group and an alkoxy-CO- group, wherein the "acyl" and "alkoxy" include, for example, the "acyl groups" and "alkoxy groups" mentioned above. Preferred specific examples, and the types and numbers of substituents are also the same as above.

Examples of unsaturated carbon compounds that satisfy the above conditions include C₂-C₂₀ unsaturated carbon compounds, such as decene, cyclododecene, and dodecine.

In the production method of the present disclosure, the amount of the organic compound used as a reaction substrate is not particularly limited. However, from the standpoints of, for example, the reaction conversion rate, selectivity of the target product, yield, and efficiency at the initial stage of the reaction, the amount is preferably 0.1 to 100 mol, more preferably 0.1 to 50 mol, even more preferably 0.1 to 15 mol, and particularly preferably 0.1 to 10 mol, per liter of the organic solvent that can be used in the reaction. Since IF₅ used in the present disclosure is a strong oxidizing agent, it is expected from common technical knowledge that increasing the amount of organic compound will also generate a large amount of decomposition product and it is assumed that the selectivity will decrease; and therefore, it is expected that the yield will also decrease. In spite of that, in the present disclosure, even if the amount of organic compound is increased, the target product is easily obtained with a high yield, and the reaction can easily proceed efficiently from the initial stage of the reaction.

### (2-2) Fluorinating Composition (Composition of the Present Disclosure)

The fluorinating composition used in the production method of the present disclosure contains IF₅, iodine (I₂), and an amine. The fluorinating composition contains 0.12 mol or less of the iodine per mole of the IF₅. Since this fluorinating composition is a composition of the present disclosure, the following description applies to all the compositions of the present disclosure.

Although it is not excluded that such a fluorinating composition (the composition of the present disclosure) is in the form of a solid, a liquid composition is preferred from the standpoints of, for example, the reaction conversion rate, selectivity of the target product, yield, efficiency at the initial stage of the reaction, economic efficiency, etc.

The fluorinating composition (the composition of the present disclosure) is usable in the production method of the present disclosure. The details thereof can be understood based on common general technical knowledge by referring to the explanation of the production method of the present disclosure in addition to the following description.

### IF₅

In the production method of the present disclosure, IF₅ may form a complex with at least one member selected from the group consisting of amines described below, acids described below, and non-amine bases described below. In both of the case where a complex is formed and the case where no complex is formed, a fluorinated organic compound can be efficiently synthesized.

In the production method of the present disclosure, the amount of IF₅ used is not particularly limited. However, from the standpoints of, for example, the reaction conversion rate, selectivity of the target product, yield, and efficiency at the initial stage of the reaction, the amount of IF₅ is preferably 0.1 to 100 mol, more preferably 0.1 to 50 mol, and even more preferably 0.1 to 15 mol, per liter of the organic solvent that can be used in the reaction. Since IF₅ used in the present disclosure is a strong oxidizing agent, it is expected from common technical knowledge that increasing the amount of organic compound will also generate a large amount of decomposition product and is assumed that the selectivity will decrease; and therefore, it is expected that the yield will also decrease and the efficiency at the initial stage of the reaction will also be reduced. In spite of that, in the present disclosure, even if the amount of IF₅ is increased, the target product is easily obtained with a high yield, and the reaction can easily proceed efficiently from the initial stage of the reaction.

In the production method of the present disclosure, the amount of IF₅ used is not particularly limited. However, from the standpoints of, for example, the reaction conversion rate, selectivity of the target product, yield, and efficiency at the initial stage of the reaction, the amount of IF₅ used is preferably 0.1 to 10.0 mol, more preferably 0.3 to 5.0 mol, and even more preferably 0.5 to 3.0 mol, per mole of the organic compound that is used as a reaction substrate.

### Iodine

In the production method of the present disclosure, when an organic compound is fluorinated, the reaction conversion rate, selectivity of the target product, yield, etc., can be improved by using a small amount of iodine (I₂), not using a large amount thereof. Furthermore, since it is easy for the reaction to proceed efficiently from the initial stage of the reaction, the reaction can be completed in a short time, and the reaction can be efficiently proceed. In particular, when a large amount of iodine (I₂) is used, the reaction substrate reacts with iodine (I₂) to generate a considerable amount of an iodine by-product, which reduces the selectivity of the target product, yield, efficiency at the initial stage of the reaction, etc. However, since a small amount of iodine (I₂) is used in the present disclosure, almost no iodine by-product is generated, and the reaction conversion rate, selectivity of the target product, yield, efficiency at the initial stage of the reaction, etc., can be improved.

In the production method of the present disclosure, the amount of iodine (I₂) used is 0.12 mol or less, preferably 0.11 mol or less, more preferably 0.10 mol or less, even more preferably 0.05 mol or less, and particularly preferably 0.01 mol or less, per mole of IF₅. If the amount of iodine (I₂) used exceeds 0.12 mol per mole of IF₅, the reaction between the reaction substrate and iodine (I₂) generates a considerable amount of iodide byproduct, resulting in a decrease in selectivity of the target product, yield, etc., and the reaction cannot proceed at the initial stage of the reaction. In the production method of the present disclosure, there is no particular limitation on the lower limit of the amount of iodine (I₂) used. Even a very small amount can achieve a high selectivity and a high yield of the target product, as well as a high reaction rate. However, the lower limit of the amount of iodine (I₂) used is usually about 1.0 × 10⁻¹⁰ mol per mole of the IF₅.

In the production method of the present disclosure, the amount of iodine (I₂) to be used is not particularly limited. However, from the standpoints of improving the reaction conversion rate, selectivity of the target product, yield, and efficiency at the initial stage of the reaction, the amount of iodine (I₂) is preferably 1.0 × 10⁻¹⁰ to 0.12 mol, more preferably 5.0 × 10⁻¹⁰ to 0.11 mol, even more preferably 1.0 × 10⁻⁹ to 0.10 mol, and particularly preferably 5.0 × 10⁻⁹ to 0.05 mol, and more particularly preferably 1.0 × 10⁻⁸ to 0.01 mol per mole of the organic compound that is a reaction substrate.

### Amine

Examples of amines include aliphatic amines (e.g., aliphatic primary amines, aliphatic secondary amines, and aliphatic tertiary amines), alicyclic amines (e.g., alicyclic secondary amines and alicyclic tertiary amines), aromatic amines (e.g., aromatic primary amines, aromatic secondary amines, and aromatic tertiary amines), and heterocyclic amines; and polymer-supported amines such as polyallylamine and polyvinylpyridine.

Examples of aliphatic primary amines include methylamine, ethylamine, propylamine, butylamine, pentylamine, hexylamine, cyclohexylamine, and ethylenediamine.

Examples of aliphatic secondary amines include dimethylamine, diethylamine, dipropylamine, dibutylamine, dipentylamine, dihexylamine, and dicyclohexylamine.

Examples of aliphatic tertiary amines include trimethylamine, triethylamine, diisopropylethylamine, and N,N,N',N'-tetramethylethylenediamine.

Examples of alicyclic secondary amines include piperidine, piperazine, pyrrolidine, and morpholine.

Examples of alicyclic tertiary amines include N-methylpiperazine, N-methylpyrrolidine, 5-diazabicyclo[4.3.0]nonane-5-ene, and 1,4-diazabicyclo[2.2.2]octane.

Examples of aromatic amines include aniline, methylaniline, dimethylaniline, N,N-dimethylaniline, haloaniline, and nitroaniline.

Examples of heterocyclic amines include one or more of pyridine, pyrimidine, piperazine, quinoline, imidazole, and the like.

Among them, aliphatic amines, alicyclic amines, aromatic amines, heterocyclic amines, and polymer-supporting amine compounds are preferred, aliphatic amines are more preferred, aliphatic tertiary amines are even more preferred, trimethylamine, pyridine, and the like are particularly preferred, and triethylamine is most preferred, from the standpoints of, for example, the reaction conversion rate, selectivity of the target product, yield, and efficiency at the initial stage of the reaction.

In the production method of the present disclosure, the amount of the amine used is not particularly limited. However, from the standpoints of, for example, the reaction conversion rate, selectivity of the target product, yield, and efficiency at the initial stage of the reaction, the content of the amine in the fluorinating composition (the composition of the present disclosure) is preferably 0.5 to 20 mol, more preferably 0.8 to 10 mol, and even more preferably 0.9 to 5 mol, per mole of the IF₅.

In the production method of the present disclosure, the amount of the amine to be used is not particularly limited. However, from the standpoints of, for example, the conversion rate, selectivity of target product, yield, and efficiency at the initial stage of reaction, the amount of the amine used is preferably 0.1 to 10.0 mol, more preferably 0.3 to 5.0 mol, and even more preferably 0.5 to 3.0 mol, per mole of the organic compound as the reaction substrate.

### Acid

The production method of the present disclosure is carried out using the above-mentioned fluorinating composition (the composition of the present disclosure). From the standpoints of, for example, the reaction conversion rate, selectivity of the target product, yield, efficiency at the initial stage of the reaction, etc., it is preferable that the fluorinating composition (the composition of the present disclosure) further contains an acid.

From the standpoints of, for example, the reaction conversion rate, selectivity of the target product, yield, and efficiency at the initial stage of the reaction, the acid is preferably a Brønsted acid or a Lewis acid, or a combination thereof, and more preferably a Brønsted acid.

Lewis acids can refer to chemical species that are not acids according to the Brønsted definition, but are acids according to the Lewis definition.

Specific examples of the acid include
sulfuric acid, nitric acid, phosphoric acid, polyphosphoric acid, hydrogen fluoride (HF), fluoric acid, hydrochloric acid, hydrogen bromide, hydrogen iodide, hypochlorous acid, chlorous acid, chloric acid, perchloric acid, perbromic acid, periodic acid, and like hydrogen halides, hydrohalic acid, hypohalous acid, halous acid, halogen acid, and perhalogen acid;
fluorosulfonic acid, chlorosulfonic acid, methanesulfonic acid, ethanesulfonic acid, trifluoromethanesulfonic acid, difluoromethanesulfonic acid, trichloromethanesulfonic acid, perfluorobutanesulfonic acid, perfluorooctanesulfonic acid, benzenesulfonic acid, toluenesulfonic acid, nitrobenzenesulfonic acid, and like sulfonic acids, polystyrenesulfonic acid; fluorinated sulfonic acid resin (Nafion-H) and like polymer-supported sulfonic acids;
formic acid, acetic acid, propionic acid, chloroacetic acid, bromoacetic acid, dichloroacetic acid, trichloroacetic acid, trifluoroacetic acid, glycolic acid, lactic acid, benzoic acid, oxalic acid, succinic acid, and like mono- or poly- carboxylic acids;
SO₃, BF₃, BCl₃, B(OCH₃)₃, AlCl₃, AlBr₃, SbF₃, SbCl₃, SbF₅, PF₃, PF₅, AsF₃, AsCl₃, AsF₅, TiCl₄, NbF₅, TaF₅, and like Lewis acids or ether complexes thereof;
HBF₄, HPF₆, HAsF₆, HSbF₆, HSbCl₆, and like acids formed from Lewis acids and hydrogen halides, or ether complexes thereof; and mixtures of two or more of these compounds.

These acids can be supported on a carrier.

Examples of the carrier include SiO₂, methylated SiO₂, Al₂O₃, Al₂O₃-WB, MoO₃, ThO₂, ZrO₂, TiO₂, Cr₂O₃, SiO₂-Al2O₃, SiO₂-TiO₂, SiO₂-ZrO₂, TiO₂-ZrO₂, Al₂-B₂O₃, SiO₂-WO₃, SiO₂-NH₄F, HSO₃Cl-Al₂O₃, HF-NH₄-Y, HF-Al₂O₃, NH₄F-SiO₂-Al₂O₃, AlF₃-Al₂O₃, Ru-F-Al₂O₃, F-Al₂O₃, KF-Al₂O₃, AlPO₄, AlF₃, bauxite, kaolin, activated carbon, graphite, Pt-graphite, ion-exchange resin, metal sulfate, chloride, metals (e.g., Al), alloys (e.g., Al-Mg and Ni-Mo), and polymers (e.g., polystyrene).

From the standpoints of, for example, the reaction conversion rate, selectivity of the target product, yield, and efficiency at the initial stage of the reaction, the acid preferably contains HF. The fluorinating composition used in the production method of the present disclosure (the composition of the present disclosure) preferably contains HF in addition to IF₅, iodine, and an amine.

The amount of the acid is not particularly limited, but is preferably 0.5 to 20 mol, more preferably 0.8 to 10 mol, and even more preferably 0.9 to 5 mol, per mole of IF₅, from the standpoints of, for example, the reaction conversion rate, selectivity of the target product, yield, and efficiency at the initial stage of the reaction.

In the production method of the present disclosure, the amount of the acid used is not particularly limited, but is preferably 0.5 to 20 mol, more preferably 0.8 to 10 mol, and even more preferably 0.9 to 5.0 mol, per mole of the organic compound used as the reaction substrate, from the standpoints of, for example, the reaction conversion rate, the selectivity of the target product, the yield, and the efficiency at the initial stage of the reaction.

### Non-amine Base

The production method of the present disclosure is carried out using the above-described fluorinating composition (the composition of the present disclosure). The composition may further comprise a non-amine base.

Examples of non-amine bases include alkali metal or alkaline earth metal hydroxides, such as sodium hydroxide, potassium hydroxide, lithium hydroxide, rubidium hydroxide, cesium hydroxide, magnesium hydroxide, calcium hydroxide, and barium hydroxide;
alkali metal alkoxides such as sodium methoxide, sodium ethoxide, sodium butoxide, potassium methoxide, potassium ethoxide, potassium butoxide, lithium methoxide, and lithium ethoxide; alkali metal or alkaline earth metal hydrides such as sodium hydride, potassium hydride, lithium hydride, and calcium hydride; alkali metals such as sodium, potassium, and lithium;
alkaline earth metal oxides such as magnesium oxide and calcium oxide; and
ammonium hydroxide salts or polymer-supported ammonium hydroxide salts (e.g., AmberLite resin), such as ammonia, ammonium hydroxide, tetramethylammonium hydroxide, tetraethylammonium hydroxide, tetrabutylammonium hydroxide, octyltriethylammonium hydroxide, and benzyltrimethylammonium hydroxide.
These compounds can be used singly or in a combination of two or more.

In the production method of the present disclosure, when a non-amine base is used, the amount of the non-amine base to be used is not particularly limited. However, from the standpoints of, for example, the reaction conversion rate, selectivity of the target product, yield, and efficiency at the initial stage of the reaction, the amount is preferably 0.5 to 20 mol, more preferably 0.8 to 10 mol, and even more preferably 0.9 to 5 mol, per mole of IF₅, in terms of the non-amine base content of the fluorinating composition (composition of the present disclosure).

In the production method of the present disclosure, the amount of the non-amine base to be used is not particularly limited. However, from the standpoints of, for example, the reaction conversion rate, selectivity of the target product, yield, and efficiency at the initial stage of the reaction, the amount of the base to be used is preferably 0.1 to 10.0 mol, more preferably 0.3 to 5.0 mol, and even more preferably 0.5 to 3.0 mol, per mole of the organic compound as the reaction substrate.

### Salt

The acid may form a salt with the amine and/or non-amine base.

Examples of the "salt" include metal salts or ammonium salts of sulfuric acid or sulfonic acid, such as sodium sulfate, sodium hydrogen sulfate, potassium sulfate, potassium hydrogen sulfate, lithium sulfate, cesium sulfate, calcium sulfate, magnesium sulfate, ammonium sulfate, triethylammonium sulfate, pyridinium sulfate, trimethylpyridinium sulfate, polyallyammonium sulfate, polyvinylpyridinium sulfate, sodium methanesulfonate, ammonium methanesulfonate, tetramethylammonium methanesulfonate, potassium ethanesulfonate, lithium butanesulfonate, sodium benzenesulfonate, sodium toluenesulfonate, sodium trifluoromethanesulfonate, and sodium polystyrenesulfonate;
metal salts or ammonium salts of carboxylic acids, such as sodium formate, ammonium formate, sodium acetate, potassium acetate, lithium acetate, magnesium acetate, calcium acetate, ammonium acetate, methylammonium acetate, diethylammonium acetate, triethylammonium acetate, tetraethylammonium acetate, pyridinium acetate, sodium propionate, potassium propionate, sodium butyrate, polyallylammonium acetate, polyvinylpyridinium acetate, sodium isobutyrate, sodium valerianate, sodium nonanoate, sodium chloroacetate, sodium bromoacetate, sodium trichloroacetate, sodium trifluoroacetate, sodium glycolate, sodium lactate, sodium benzoate, sodium oxalate, sodium succinate, and sodium polyacrylate;
metal salts, such as LiBr, LiI, NaBr, NaI, KBr, KI, RbBr, RbI, CsBr, CsI, BeBr₂, BeI₂, MgBr₂, MgI₂, CaBr₂, CaI₂, SrBr₂, SrI₂, BaBr₂, BaI₂, ZnBr₂, ZnI₂, CuBr₂, CuI₂, CuBr, CuI, AgBr, AgI, AuBr, AuI, NiBr₂, NiI₂, PdBr₂, PdI₂, PtBr₂, PtI₂, CoBr₂, CoI₂, FeBr₂, FeBr₃, FeI₂, FeI₃, MnBr₂, MnI₂, CrBr₂, CrI₂, PbBr₂, PbI₂, SnBr₂, SnI₂, SnBr₄, and SnI₄;
pyridinium halide salts or ammonium halide salts, such as NH₄Br, NH₄I, MeNH₃Br, MeNH₃I, Me₄NBr, Me₄NI, Et₄NBr, Et₄NI, Bu₄NBr, Bu₄NI, PhMe₃NBr, PhMe₃NI, PhCH₂NMe₃I, pyridinium bromide, pyridinium iodide, chloropyridinium iodide, methylpyridinium iodide, cyanopyridinium iodide, bipyridinium iodide, quinolium iodide, isoquinolium iodide, N-methylpyridinium bromide, N-methylpyridinium iodide, and N-methylquinolium iodide (wherein Me represents methyl, Et represents ethyl, Bu represents n-butyl, and Ph represents phenyl);
phosphonium salts, such as Me₄PF, Me₄PBr, Me₄PI, Et₄PI, Pr₄I, Bu₄PBr, Bu₄PI, Ph₄PF, Ph₄PBr, Ph₄PI, and tetramethylphosphonium acetate (wherein Me represents methyl, Et represents ethyl, Pr represents n-propyl, Bu represents n-butyl, and Ph represents phenyl) ;
metal salts or amine salts of hydrogen halide, hypohalous acid, halous acid, halogen acid, or perhalogen acid, such as sodium fluoride, potassium fluoride, cesium fluoride, ammonium fluoride, tetraethylammonium fluoride, tetrabutylammonium fluoride, polyarylammonium fluoride, sodium chloride, ammonium chloride, sodium hypochlorite, sodium chlorite, sodium chlorate, sodium perchlorate, sodium perbromate, and sodium periodate;
metal carbonates such as sodium carbonate, potassium carbonate, lithium carbonate, sodium bicarbonate, potassium bicarbonate, lithium bicarbonate, calcium carbonate, and magnesium carbonate;
metal salts or amine salts of phosphorous acid, such as sodium phosphate, potassium phosphate, sodium hydrogen phosphate, sodium dihydrogen phosphate, ammonium phosphate, and pyridinium phosphate;
metal salts or amine salts of nitric acid, such as sodium nitrate, potassium nitrate, ammonium nitrate, and pyridinium nitrate;
metal salts or amine salts of Lewis acids and hydrogen halides, such as NaBF₄, KBF₄, LiBF₄, NaSbF₆, NaAsF₆, NaPF₆, NH₄BF₄, NH₄SbF₆, and NH₄PF₆; and
room-temperature molten salts having fluoride anions or HF, such as (C₂H₅)₄NF, 1-ethyl-3-methylimidazolium fluoride, (C₂H₅)₃N-(HF)ₙ, (C₂H₅)₄NF-(HF)ₙ, (n-C₄H₉)₃N-(HF)ₙ, (n-C₄H₉)₄NF-(HF)ₙ, BF₃·Et₂O-(HF)ₙ (wherein n is 1 to 20, and Et represents ethyl).

The "salt" may be used singly, or as a mixture or a combination of two or more of salts.

The amount of the salt to be used can be determined, based on the amounts of the above-mentioned amine, acid, and non-amine base used, and can be adjusted appropriately.

### Other additives

If desired, the fluorinating composition (the composition of the present disclosure) may further contain additives, such as non-iodine halogens, interhalogen compounds, and polyhalides.

Examples of the "non-iodine halogens" include bromine and chlorine. Among such halogens, bromine is preferred from the standpoints of, for example, the reaction conversion rate, selectivity of the target product, yield, and efficiency at the initial stage of the reaction.

The "interhalogen compounds" means non-IF₅ interhalogen compounds. Examples include ClF, BrF, ICl, IBr, I₂Cl₆, and ICl₃.

Examples of the "polyhalides" include LiCl₄I, NaCl₄I, KCl₄I, CsCl₄I, RbCl₄I, Me₄NCl₄I, Et₄NCl₄I, Pr₄NCl₄I, Bu₄NCl₄I, PhNMe₃Cl₄I, PhCH₂NMe₃Cl₄I, Me₃SCl₄I, Cl_{g}IP, KCl₃I₂, Me₄NCl₃I₂, 2,2'-bipyridinium µ-chlorodichlorodiiodate, 2,2'-biquinolinium µ-chlorodichlorodiiodate, KCl₂I, Me₄NCl₂I, Me₄NClI₂, Et₄NCl₃, Ph₄AsCl₃, KClF₂, Me₄NClF₄, CsClF₄, CsCl₃FI, KBrClI, NH₄BrClI, Me₄NBrClI, Me₄NBrCl₂, Bu₄NBrCl₂, Me₄NBrCl₂I₂, CsBrFI, NaBrF₂, KBrF₂, CsBrF₄, Me₄NBrF₄, CsBrF₆, Me₄NBrF₆, Et₄NBr₆Cl, CsBr₃, Me₄NBr₃, Et₄Br₃, Bu₄NBr₃, PhCH₂NMe₃Br₃, pyridinium tribromide, Br₇P, CsBrI₂, Me₄NBrI₂, Me₄NBrI₄, Me₄NBrI₆, KBr₂Cl, Me₄NBr₂Cl, Bu₄NBr₂Cl, KBr₂I, Me₄NBr₂I, Bu₄NBr₂I, 2,2'-bipyridinium µ-bromodibromodiiodate, NaF₂I, KF₂I, CsF₄I, CsF₆I, CsF₈I, KI₃, CsI₃, Me₄NI₃, Et₄NI₃, Pr₄NI₃, Bu₄NI₃, pyridinium triiodide, Me₄NI₅, Et₄NI₇, Me₄NI₉, Me₄PBr₃, Me₄PI₃, Me₄PIBr₂, Me₄PICl₂, Et₄PI₃, Bu₄PI₃, Ph₄PI₃, Ph₄PBr₃, and Ph₄PIBr₂ (wherein Me represents methyl, Et represents ethyl, Pr represents n-propyl, Bu represents n-butyl, and Ph represents phenyl) .

The "additives" may be used singly, or as a mixture or a combination of two or more additives.

When the additives described above are used, the additives can be used in amounts within the range that does not impair the effects of the present disclosure, and can be appropriately adjusted. However, when a non-iodine halogen is used as an additive, the amount of the halogen used is preferably equivalent to or less than the amount of iodine used from the viewpoint of minimizing the generation of by-products.

### Organic Solvent

The production method of the present disclosure does not exclude the reaction being carried out in the absence of a solvent. However, from the standpoints of, for example, the reaction conversion rate, selectivity of the target product, yield, and efficiency at the initial stage of the reaction, it is preferable to carry out the reaction in the presence of an organic solvent. That is, the fluorinating composition (the composition of the present disclosure) is preferably a liquid composition using an organic solvent.

The organic solvent is preferably an aprotic organic solvent from the standpoints of, for example, the selectivity of the target product, yield, and efficiency at the initial stage of the reaction.

From the standpoints of, for example, the selectivity of the target product, yield, and efficiency at the initial stage of the reaction, the organic solvent preferably used in the production method of the present disclosure is, for example, aliphatic solvents (aliphatic hydrocarbons), such as pentane, hexane, heptane, cyclohexane, and petroleum ether;
halogenated aliphatic solvents (halogenated hydrocarbons), such as dichloromethane, dichloroethane, chloroform, fluorotrichloromethane, 1,1,2-trichlorotrifluoroethane, 2-chloro-1,2-dibromo-1,1,2-trifluoroethane, 1,2-dibromohexafluoropropane, 1,2-dibromotetrafluoroethane, 1,1-difluorotetrachloroethane, 1,2-difluorotetrachloroethane, heptafluoro-2,3,3-trichlorobutane, 1,1,1,3-tetrachlorotetrafluoropropane, 1,1,1-trichloropentafluoropropane, 1,1,1-trichlorotrifluoroethane, and polychlorotrifluoroethylene;
ester solvents such as methyl formate, ethyl formate, methyl acetate, ethyl acetate, propyl acetate, methyl propionate, γ-butyrolactone, and propylene carbonate;
nitrile solvents, such as acetonitrile and propionitrile;
aromatic solvents (aromatic hydrocarbons), such as benzene, chlorobenzene, toluene, dichlorobenzene, fluorobenzene, and nitrobenzene;
ether solvents, such as diethyl ether, dipropyl ether, tetrahydrofuran, cyclopentyl methyl ether (CPME), and methyl tertiary butyl ether (MTBE);
ketone solvents, such as acetone;
amide solvents, such as N,N-dimethylformamide (DMF), N,N-diethylformamide, N,N-dimethylacetamide, 1-methyl-2-pyrrolidone, 1,3-dimethyl-2-imidazolidinone (DMI), tetramethylurea, 1,3-dimethylpropyleneurea, and hexamethylphosphoramide (HMPA);
dimethyl sulfoxide (DMSO); and
nitromethane.

These solvents may be used singly or in any combination of two or more (e.g., as mixed solvents).

Among these, aliphatic solvents, halogenated aliphatic solvents, ester solvents, nitrile solvents, aromatic solvents, ether solvents, ketone solvents, amide solvents, and the like are preferred; aliphatic solvents, halogenated aliphatic solvents, nitrile solvents, aromatic solvents, and the like are more preferred; and halogenated aliphatic solvents, nitrile solvents, aromatic solvents, and the like are even more preferred, from the standpoints of, for example, the reaction conversion rate, selectivity of the target product, yield, and efficiency at the initial stage of the reaction.

### Reaction Conditions etc.

The reaction temperature for fluorination in the production method of the present disclosure is not particularly limited. However, from the standpoints of, for example, the reaction conversion rate, selectivity of the target product, yield, and efficiency at the initial stage of the reaction, the reaction temperature is, for example, preferably -20 to 120°C, more preferably -10 to 100°C, even more preferably 0 to 90°C, and particularly preferably 5 to 80°C.

The reaction time for fluorination in the production method according to the present disclosure may vary depending on, for example, the reaction substrate (organic compound) and the target product. From the standpoints of, for example, the reaction conversion rate, selectivity of the target product, yield, efficiency at the initial stage of the reaction, and economic efficiency, the reaction time is, for example, preferably 3 minutes to 80 hours, more preferably 5 minutes to 60 hours, even more preferably 30 minutes to 40 hours, particularly preferably 1 to 30 hours, and most preferably 2 to 24 hours.

The atmosphere in which the fluorination is carried out in the present disclosure is not particularly limited, but an inert gas atmosphere is preferred. The inert gas may be nitrogen, helium, argon, or the like.

The reaction pressure of the fluorination in the present disclosure is not particularly limited. However, from the standpoints of, for example, the reaction conversion rate, selectivity of the target product, yield, efficiency at the initial stage of the reaction, and economic efficiency, the reaction pressure is preferably 0 kPa or more, more preferably 10 kPa or more, even more preferably 20 kPa or more, and particularly preferably 30 kPa or more. There is no particular upper limit on the reaction pressure, and it is usually about 2 MPa. In this disclosure, pressure refers to gauge pressure, unless otherwise specified.

The shape and structure of the reactor usable in the fluorination of the present disclosure are not particularly limited as long as the reactor can withstand the temperature and pressure described above. Examples of the reactor include vertical reactors, horizontal reactors, and multi-tube reactors. Examples of materials for the reactor to be used include stainless steel, iron, nickel, iron-nickel alloy, Hastelloy, and fluororesin.

After completion of the fluorination reaction, a purification treatment can be carried out according to a usual method, if necessary, to obtain a fluorinated organic compound.

The number (molar number) of fluorine atoms introduced by the reaction of the present disclosure per mole of the reaction substrate is not particularly limited, and can be, for example, 0.65 to 20 mol, 0.70 to 10 mol, 0.75 to 7 mol, or 0.80 to 5 mol.

### Examples

Examples are given below to clarify the features of the present disclosure. The present disclosure is not limited to these examples.

In the following Examples, the reactions were carried out under a nitrogen atmosphere.

The meaning of the symbol and abbreviation used in the examples is as follows.
Et₃N: triethylamine.

In the following Examples, the iodine content of each fluorinating composition was determined in the following manner.
1. A fluorinating composition (about 7 g) is added dropwise to ice water (30 g).
2. Iodine is extracted using chloroform.
3. The obtained chloroform layer is washed with a sodium bicarbonate solution and water.
4. A 0.1 mol/L aqueous Na₂S₂O₃ solution (15 g) is added to the chloroform layer and vigorously stirred at room temperature for 10 minutes.
5. An aqueous layer is separated.
6. After the aqueous layer is appropriately diluted with water, the iodine content is quantified in terms of iodide ion by ion chromatography.

The iodine content of a fluorinating composition prepared by adding iodine (66.0 mg, 0.26 mmol) to IF₅-Et₃N-3HF (1.0 g, 2.62 mmol) was quantified by the procedure described above. The iodine content was found to be 65.8 mg, which confirmed that the analytical method was reliable.

### Example 1

Dichloromethane (4.0 mL) was placed in a reactor. IF₅-Et₃N-3HF (460 mg, 1.2 mmol) and iodine that was diluted with dichloromethane (1 mL, containing 0.0001 mg, i.e., 0.0000004 mmol of iodine) were then added with stirring. Thereafter, ethyl 3-oxobutanoate (130 mg, 1.0 mmol) was added. The resulting mixture was reacted at room temperature. After 3 hours, F-NMR confirmed that 26% of ethyl 2-fluoro-3-oxobutanoate and 1% of ethyl 2-difluoro-3-oxobutanoate were produced.

### Example 2

Dichloromethane (4.0 mL) was placed in a reactor. IF₅-Et₃N-3HF (460 mg, 1.2 mmol) and iodine (1.5 mg, 0.0059 mmol) were then added with stirring. Thereafter, ethyl 3-oxobutanoate (130 mg, 1.0 mmol) was added. The resulting mixture was reacted at room temperature. After 3 hours, F-NMR confirmed that 20% of ethyl 2-fluoro-3-oxobutanoate and 1% of ethyl 2-difluoro-3-oxobutanoate were produced.

### Example 3

Dichloromethane (4.0 mL) was placed in a reactor. IF₅-Et₃N-3HF (460 mg, 1.2 mmol) and iodine (15.2 mg, 0.06 mmol) were then added with stirring. Thereafter, ethyl 3-oxobutanoate (130 mg, 1.0 mmol) was added. The resulting mixture was reacted at room temperature. After 3 hours, F-NMR confirmed that 18% of ethyl 2-fluoro-3-oxobutanoate and 2% of ethyl 2-difluoro-3-oxobutanoate were produced.

### Example 4

Toluene (3.0 mL) was placed in a reactor. IF₅-Et₃N-3HF (460 mg, 1.2 mmol) and iodine diluted with dichloromethane (1 mL, containing 0.0001 mg, i.e., 0.0000004 mmol of iodine) were added with stirring. Thereafter, O-n-decyl S-methyl dithiocarbonate (297.6 mg, 1.2 mmol) was added. The resulting mixture was allowed to react at room temperature. After 10 hours, F-NMR confirmed that 20% of n-decyl trifluoromethyl ether (CF₃ form) and 49% of methyl n-decyloxydifluoromethyl sulfide (CF₂ form) were produced.

### Example 5

Toluene (3.0 mL) was placed in a reactor. IF₅-Et₃N-3HF (460 mg, 1.2 mmol) and iodine (14.3 mg, 0.057 mmol) were then added with stirring. Thereafter, O-n-decyl S-methyldithiocarbonate (297.6 mg, 1.2 mmol) was added. The resulting mixture was allowed to react at room temperature. After 10 hours, F-NMR confirmed that 22% of n-decyl trifluoromethyl ether (CF₃ form) and 51% of methyl n-decyloxydifluoromethyl sulfide (CF₂ form) were produced.

### Comparative Example 1

Dichloromethane (4.0 mL) was placed in a reactor. IF₅-Et₃N-3HF (460 mg, 1.2 mmol) and iodine (151.8 mg, 0.6 mmol) were then added with stirring. Thereafter, ethyl 3-oxobutanoate (130 mg, 1.0 mmol) was added. The resulting mixture was allowed to react at room temperature. After 3 hours, F-NMR confirmed that 0.1% of ethyl 2-fluoro-3-oxobutanoate was produced. Ethyl 2-difluoro-3-oxobutanoate was not produced.

### Comparative Example 2

Dichloromethane (4.0 mL) was placed in a reactor. IF₅-Et₃N-3HF (460 mg, 1.2 mmol) was then added with stirring. Thereafter, ethyl 3-oxobutanoate (130 mg, 1.0 mmol) was added. The resulting mixture was allowed to react at room temperature. After 3 hours, F-NMR confirmed that 11% of ethyl 2-fluoro-3-oxobutanoate and 6% of ethyl 2-difluoro-3-oxobutanoate were produced.

### Example 6 and Comparative Example 3

Monoglyme (1.0 mL) and acetonitrile (2.0 mL) were placed in a reactor. While stirring, IF₅-Et₃N-3HF (1264 mg, 3.3 mmol) was then added to a reactor and iodine (Example 6: 126 mg, 0.33 mmol; Comparative Example 3: no addition) was added to a reactor with stirring. Thereafter, O-(3,4,5-trifluorophenyl) 4"-butyl-2',3,5-trifluoro-[1,1':4'1"-terphenyl]-4-carbothioate (1.59 g, 3.0 mmol) was added. The resulting mixture was allowed to react at 50°C. The table below shows the yield of 4"-butyl-4-(difluoro(3,4,5-trifluorophenoxy)methyl)-2',3,5-trifluoro-1,1':4',1"-terphenyl at each time point. The results confirmed that the addition of iodine can shorten the time required for the reaction to start.

**Table 1**

| Yield at each time point | | | | | |
|---|---|---|---|---|---|
| Reaction time (min) | 5 | 30 | 90 | 120 | 180 |
| Example 6 | 65 | 88 | 93 | 97 | |
| Comparative Example 3 | 1 | 10 | | | 83 |

### Comparative Example 4

Toluene (10.0 mL) was placed into a reactor. IF₅-Et₃N-3HF (1.54 mg, 4.03 mmol) was then added with stirring. Thereafter, O-n-decyl S-methyl dithiocarbonate (1.0 g, 4.03 mmol) was added. The resulting mixture was allowed to react at room temperature. After 10 hours, F-NMR confirmed that 5% of n-decyl trifluoromethyl ether (CF₃ form) and 50% of methyl n-decyloxydifluoromethyl sulfide (CF₂ form) were produced.

### Industrial Applicability

According to the present invention, for example, a production method capable of efficiently synthesizing a fluorinated organic compound, and a composition capable of efficiently fluorinating an organic compound can be provided.

## Claims

1. A method for producing a fluorinated organic compound, comprising fluorinating an organic compound in the presence of a fluorinating composition,
the fluorinating composition comprising IF₅, iodine, and an amine,
the fluorinating composition comprising the iodine in an amount of 0.12 mol or less per mole of the IF₅.

2. The method according to claim 1, wherein the content of the iodine is 0.11 mol or less per mole of the IF₅.

3. The method according to claim 1 or 2, wherein the amine comprises at least one amine selected from the group consisting of an aliphatic amine, an alicyclic amine, an aromatic amine, a heterocyclic amine, and a polymer-supported amine.

4. The method according to any one of claims 1 to 3, wherein the amine comprises an aliphatic amine.

5. The method according to claim 3 or 4, wherein the aliphatic amine comprises an aliphatic tertiary amine.

6. The method according to claim 5, wherein the aliphatic tertiary amine comprises triethylamine.

7. The method according to any one of claims 1 to 6,
wherein the fluorinating composition further comprises an acid.

8. The method according to claim 7, wherein the acid comprises a Brønsted acid and/or a Lewis acid.

9. The method according to claim 7 or 8, wherein the acid comprises HF.

10. A composition comprising IF₅, iodine, and an amine,
the composition comprising the iodine in an amount of 0.12 mol or less per mole of the IF₅.

11. The composition according to claim 10, wherein the content of the iodine is 0.11 mol or less per mole of the IF₅.

12. The composition according to claim 10 or 11, wherein the amine comprises at least one amine selected from the group consisting of an aliphatic amine, an alicyclic amine, an aromatic amine, a heterocyclic amine, and a polymer-supported amine.

13. The composition according to any one of claims 10 to 12, wherein the amine comprises an aliphatic amine.

14. The composition according to claim 12 or 13, wherein the aliphatic amine comprises an aliphatic tertiary amine.

15. The composition according to claim 14, wherein the aliphatic tertiary amine comprises triethylamine.

16. The composition according to any one of claims 10 to 15, further comprising an acid.

17. The composition according to claim 16, wherein the acid comprises a Brønsted acid and/or a Lewis acid.

18. The composition according to claim 16 or 17, wherein the acid comprises HF.

19. A reagent for fluorinating an organic compound, the reagent comprising IF₅, iodine, and an amine,
the reagent comprising the iodine in an amount of 0.12 mol or less per mole of the IF₅.
